# FASCICULE DE BREVET EUROPEEN

(11) **EP 1 284 983 B1**
(45) Date de publication et mention de la délivrance du brevet: **23.11.2005**
(21) Numéro de dépôt: 01931825.2
(22) Date de dépôt: 09.05.2001
(51) Int. Cl.: C07H 17/08, A61K 31/7048, A61P 31/04

(54) **PROCEDE UTILE POUR TRANSFORMER LA FONCTION CARBONYLE EN POSITION 4" DU MOTIF CLADINOSE D'UN AZA MACROLIDE EN UN DERIVE AMINE**
VERFAHREN ZUR UMWANDLUNG VON EINEM 4'' CLADINOSE CARBONYL IN EIN AZA-MAKROLID ZU EINEM AMINDERIVAT
METHOD USED FOR TRANSFORMING THE CARBONYL FUNCTION IN POSITION 4'' OF A CLADINOSE UNIT OF AN AZA MACROLIDE INTO AN AMINE DERIVATIVE

(30) Priorité: 30.05.2000 FR 0006942
(43) Date de publication de la demande: 26.02.2003
(73) Titulaire: MERIAL, 69002 Lyon (FR)
(72) Inventeur: DHAINAUT, Jildaz, F-01800 Rigneux le Franc (FR); LEON, Patrick, F-69160 Tassin (FR); LHERMITTE, Frédéric, F-69360 Saint Symphorien D'Ozon (FR); ODDON, Gilles, F-69003 Lyon (FR)
(74) Mandataire: Nargolwalla, Cyra
(86) Numéro de dépôt international: PCT/FR2001/001398
(87) Numéro de publication internationale: WO 2001/092281

(56) Documents cités:
- EP-A- 0 508 699
- K SHANKARAN ET AL: "Preparation and Activities of 4-Epi and 4-deoxy-4-Amino Analogs Derived From 9-Deoxo-8a-Aza-8a-Homoerythromycin A" BIOORGANIC & MEDICINAL CHEMISTRY LETTERS,GB,OXFORD, vol. 4, no. 9, 1994, pages 1111-1116, XP002118137 ISSN: 0960-894X

## Description

La présente invention a pour objet un procédé notamment utile pour transformer la fonction carbonyle en position 4" du motif cladinose d'un aza-macrolide en un dérivé aminé.

La présente invention concerne plus particulièrement le domaine des antibiotiques macrolides de type érythromycine et plus particulièrement leurs dérivés aza-macrolides qui font l'objet du brevet EP 508 699 et répondent à la formule générale suivante : 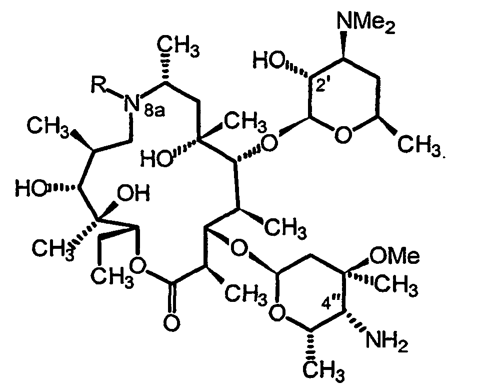 dans laquelle R représente un atome d'hydrogène, un groupement alkyle en C₁-C₁₀, alkényle en C₂-C₁₀ ou arylesulfonyle en C₆-C₁₂, le cas échéant substitués.

Ces composés sont obtenus à partir de l'érythromycine et leur synthèse implique deux étapes majeures :
- la création du macrocycle 8a-azalide au départ de l'oxime (Z) qui subit un réarrangement stéréospécifique de Beckmann et
- la modification du groupe cladinose en position 4" consistant en la transformation du 4"(S)-OH en 4"(R)-NH₂ c'est-à-dire avec inversion de configuration que l'on peut illustrer comme suit :

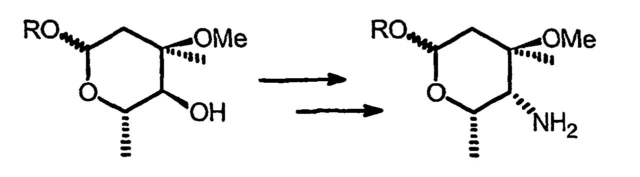

En fait, la voie actuellement retenue pour assurer cette transformation de la fonction 4"(S)-OH en 4"(R)-NH₂ n'est pas totalement appropriée à une production à l'échelle industrielle.

Elle implique successivement une oxydation de la fonction hydroxyle en position 4" en fonction cétonique puis la transformation de cette cétone en oxime qui par réduction conduit à un mélange d'environ 1 pour 1 du dérivé aminé attendu et de son épimère en 4". Les isomères obtenus à l'issue de cette voie de synthèse, sont obtenus avec un rendement faible de l'ordre de 20 % et sont en outre difficilement séparables par chromatographie. C'est ainsi que pour un rendement brut de réaction de l'ordre de 20 % on obtient seulement environ 7 % du dérivé aminé avec inversion de configuration.

La présente invention a précisément pour objet de proposer une nouvelle voie d'accès à ces dérivés aminés en position 4" avec un rendement satisfaisant.

Plus précisément, la présente invention a pour objet un procédé de préparation d'un composé de formule générale I 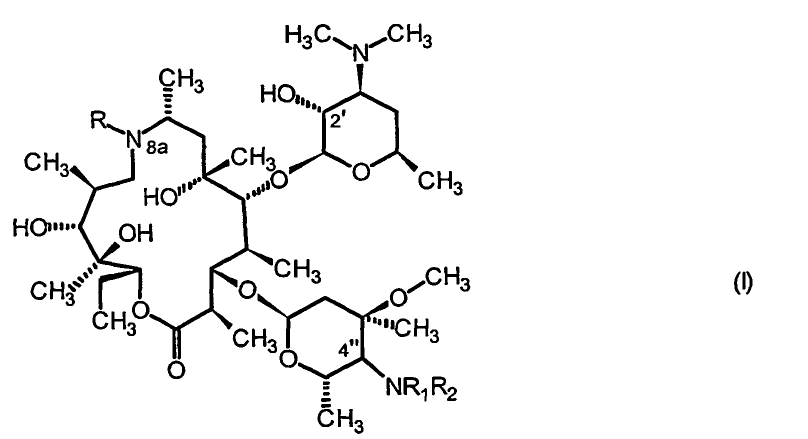 dans laquelle :
- R est un atome d'hydrogène, un groupement alkyle en C₁-C₁₀, alkényle en C₂-C₁₀ ou arylsulfonyle en C₆-C₁₂, le cas échéant substitués et
- R₁ et R₂, identiques ou différents, représentent
   - un atome d'hydrogène,
   - un groupement alkyle en C₁-C₁₀ éventuellement substitué par un ou plusieurs groupements aryles le cas échéant substitués, ou un groupement aryle en C₆-C₁₂ le cas échéant substitué,
par amination réductrice d'un composé de formule générale II 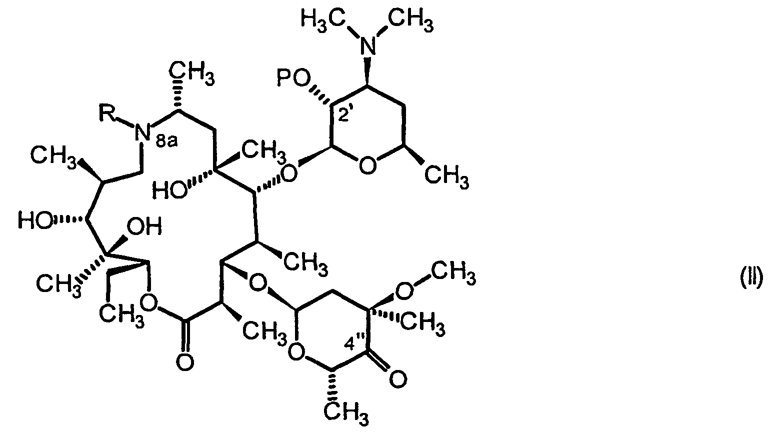 avec :
- R tel que défini en formule générale I, et
- P représentant un atome d'hydrogène ou un groupement protecteur,
caractérisé en ce qu'il comprend :
- la mise en présence dudit composé de formule générale II avec au moins un réactif azoté et un acide de Lewis aprotique dans des conditions favorables à la transformation de la fonction carbonyle en 4",
- la réduction du mélange résultant à l'aide d'un agent réducteur, et
- éventuellement la déprotection de la fonction hydroxyle en position 2',
pour conduire au composé de formule générale I attendu.

On obtient à l'issue dudit procédé les deux formes isomériques R et S du dérivé 4" aminé attendu. Toutefois, la diastéréosélectivité est telle que la forme 4" R est obtenue généralement majoritairement. Comme il ressort des exemples soumis ci-après, le procédé revendiqué peut avantageusement conduire à un mélange 4"R/4"S avec un rapport molaire de 60/40 et pouvant atteindre jusqu'à 90/10.

En conséquence, le procédé revendiqué s'avère particulièrement intéressant pour obtenir ce dérivé (4"R)-amino dont la séparation avec la forme (4"S)-amino obtenue minoritairement peut alors être opérée par les techniques connues de l'homme de l'art.

Dans le cadre de la présente invention, on fait donc réagir un composé de formule générale II avec un agent azoté et un acide de Lewis aprotique dans des conditions suffisantes pour permettre la transformation de la fonction carbonyle en position 4". Lorsque cette transformation est achevée, on réduit directement le mélange résultant pour obtenir le composé de formule générale 1.

Avantageusement, le procédé revendiqué ne nécessite pas d'étape intermédiaire et permet l'enchaînement des deux réactions au sein d'un même milieu réactionnel.

Comme réactif azoté, on peut notamment utiliser un composé choisi parmi l'ammoniac, les sels d'ammonium comme l'acétate d'ammonium, le chlorhydrate d'ammonium, les amines primaires NH₂R_{A}, les amines secondaires NH(R_{A})₂ avec les radicaux R_{A} identiques ou différents représentant un groupement alkyle en C₁ à C₁₀, aryle en C₆ à C₁₂ ou silyle de type SiR_{B}R_{C}R_{D} dans lequel les groupements R_{B}, R_{C} et R_{D} identiques ou différents, peuvent être un groupement alkyle ou aryle.

Conviennent tout particulièrement à l'invention la benzylamine, l'ammoniac et l'hexaméthyldisilazane.

Le réactif azoté peut être utilisé à raison de 1 à 30 équivalents par rapport au composé de formule générale II et de préférence à raison de 1 à 10 équivalents.

Conviennent tout particulièrement comme acide de Lewis, les complexes organométalliques des éléments de la colonne IVB, IIIA ou IIB du tableau de la classification périodique et en particulier ceux à base de titane, de zinc et d'aluminium. Les substituants sur ces complexes peuvent être de type alcoxy, acyloxy, sulfonate, halogéné, base de Schiff, acétylacétonate ou ligand π-donneur tel que cyclopentadiènyle.

Conviennent ainsi à l'invention les composés suivants : l'isopropoxyde de titane (IV), l'isopropoxyde d'aluminium (III), l'isopropoxyde bis (acétyl acétonate) de titane (IV), le trifluoroacétate de zinc (II).

Cet acide de Lewis peut ainsi être utilisé à raison de 1 à 30 équivalents par rapport au composé de formule générale II et plus préférentiellement à raison de 1 à 10 équivalents.

Comme discuté précédemment, l'acide de Lewis et le réactif azoté sont dans une première étape mis en présence du composé de formule générale II.

Il est ainsi possible de faire réagir :
- l'acide de Lewis et le composé de formule générale II avant d'introduire le réactif azoté,
- l'acide de Lewis avec le réactif azoté puis le composé de formule générale II ou encore
- simultanément le réactif azoté, le composé de formule générale II et l'acide de Lewis.

Quel que soit l'ordre de mise en présence retenu, l'agent réducteur n'est lui ajouté qu'à partir du moment où la transformation de la fonction cétonique en 4" est achevée.

Avantageusement, on procède directement à la réduction du milieu réactionnel à l'aide d'un agent réducteur.

Conviennent tout particulièrement à l'invention comme agent réducteur les hydrures métalliques. De préférence, il s'agit d'un hydrure d'aluminium ou de bore et plus préférentiellement de bore substitué ou non.

Peuvent ainsi être utilisés comme borohydrures substitués, les borohydrures mono-, di- ou tri- substitués par :
- des acides mono- ou di- carboxyliques comme RCO₂H avec R représentant un groupement alkyle ou aryle le cas échéant substitué,
- des alcools de type ROH avec R tel que défini ci-dessus, ou
- des diols-1,2, 1,3 ou 1,4 et associés à un contre cation, de nature soit alcaline tel Li, Na, K, soit organique de type ammonium quaternaire ou encore métallique tel zinc, calcium, zirconium.

Avantageusement, il s'agit d'un borohydrure différent d'un cyanoborohydrure.

Conviennent tout particulièrement à l'invention les borohydrures de sodium, de lithium et de zinc, ou le dibenzoyloxyborohydrure de sodium.

L'agent réducteur est utilisé en quantité suffisante pour former le composé de formule générale III :

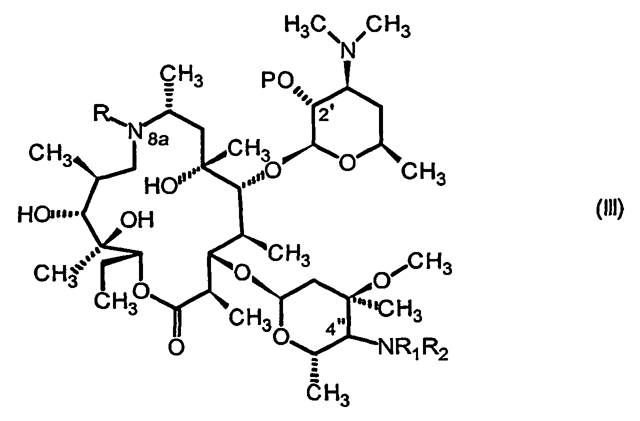

Généralement, la quantité utilisée varie de 1 à 10 équivalents et plus préférentiellement de 1 à 5.

L'ensemble du procédé est généralement réalisé au sein d'un solvant organique.

Ce solvant peut être notamment choisi parmi les hydrocarbures aromatiques comme le toluène, les solvants halogénés tel le dichlorométhane, les alcools comme le méthanol, les nitriles comme l'acétonitrile, les éthers comme le THF et les sulfoxides comme le DMSO.

En ce qui concerne les autres paramètres réactionnels, à savoir température et durée de réaction, leur ajustement relève des compétences de l'homme de l'art. La réaction peut ainsi être conduite à une température comprise entre -30°C et la température de reflux du solvant et cette température réactionnelle peut varier en cours de réaction.

En ce qui concerne le composé de formule générale II, il est généralement obtenu au préalable à partir du composé de formule générale IV : 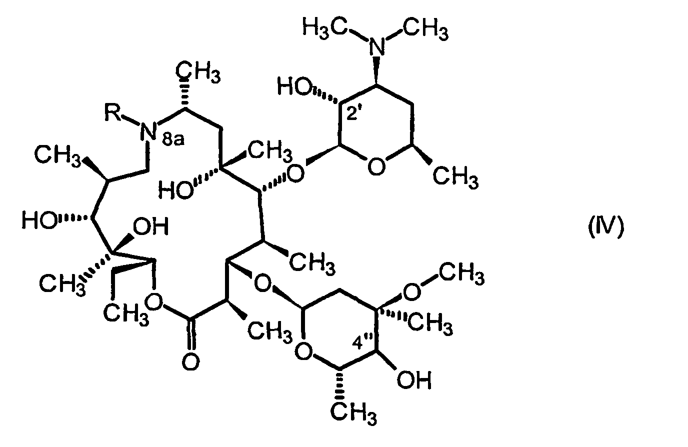 par protection de la fonction hydroxyle en position 2' dudit composé puis oxydation de la fonction hydroxyle en position 4".

La protection est réalisée de manière classique à l'aide d'un groupement protecteur de fonction hydroxyle conventionnel tels ceux figurants dans « Protective groups in organic synthesis » Second Edition Theodora W. Greene, P. G. Wuts, Wiley Intersciences p10-142. Les protocoles de réalisation des opérations, protection et déprotection, sont également décrits dans l'ouvrage référencé ci-dessus.

En ce qui concerne l'oxydation, elle peut être réalisée selon le protocole décrit dans EP 508 699.

A l'issue de la réduction selon le procédé revendiqué, on procède donc si nécessaire à la déprotection de la fonction en 2'.

Selon des variantes préférées de l'invention, on utilise à titre d'acide de Lewis, l'isopropoxyde de titane ou d'aluminium. En ce qui concerne le réactif azoté associé, il est de préférence choisi parmi l'ammoniac, l'hexaméthyldisilazane et la benzylamine. Dans ces conditions, le solvant utilisé est de préférence choisi parmi le dichlorométhane, le tétrahydrofurane et le toluène.

L'agent réducteur préféré est le borohydrure de sodium ou de lithium.

Selon une première variante de l'invention, le composé de formule II en solution dans un solvant organique, de préférence le toluène ou le dichlorométhane, est ajouté à un mélange de l'acide de Lewis tel l'isopropoxyde de titane IV ou l'isopropoxyde d'aluminium III avec le réactif azoté, comme la benzylamine, l'hexaméthyldisilazane ou l'ammoniac, et l'ensemble est maintenu agité à température ambiante et éventuellement chauffé à reflux du milieu réactionnel.

Dans une seconde variante, on met en présence l'acide de Lewis, tel l'isopropoxyde de titane (IV), avec le composé de formule II au sein d'un solvant organique comme le THF ou le toluène et l'on procède ensuite à l'addition lente du réactif azoté, de préférence l'hexaméthyldisilazane. L'ensemble est maintenu agité à température ambiante et éventuellement chauffé à reflux du milieu réactionnel.

Selon une troisième variante, le composé de formule générale II est mélangé avec le réactif azoté de préférence l'ammoniac dans un solvant organique. L'acide de Lewis, de préférence le diisopropoxyde bis(acétylacétonate) de titane en solution dans le même solvant organique, y est ensuite ajouté et l'ensemble est maintenu agité à température ambiante et éventuellement chauffé à reflux du milieu réactionnel.

Quelle que soit la variante considérée, la réduction est réalisée consécutivement par ajout direct de l'agent réducteur dans le milieu réactionnel. On laisse la réduction s'achever puis l'on procède à une hydrolyse du milieu réactionnel puis à au moins une extraction.

On procède ensuite si nécessaire à la déprotection du composé de formule générale III de manière à obtenir le composé de formule générale I qui est ensuite isolé selon un mode opératoire conventionnel qui implique généralement des opérations d'extraction, de lavage puis de séchage.

Les exemples figurant ci-après sont présentés à titre illustratif et non limitatif de la présente invention.

### EXEMPLE 1

### Synthèse de la 2'-O-acetyl-4"-deoxy-4"-N-benzylamino-9-deoxo-8a-aza-8a-méthyl-8a-homoérythromycine A

Une solution d'isopropoxyde de titane (IV) (0,3 ml, 1 mmol, 8 équiv.) et de benzylamine (100 mg, 0,93 mmol, 7,4 équiv.) est agitée 1h à température ambiante. Une solution de 2'-O-acétyl-4"-deoxy-4"-oxo-9-deoxo-8a-aza-8a-méthyl-8a-homoérythromycine A (100 mg, 0,12 mmol) dans du THF (0,5 ml) est additionnée. Le mélange réactionnel est agité à température ambiante pendant 96h puis est additionnée une solution 2M de borohydrure de lithium dans du THF (0,25 ml, 0,5 mmol, 4 équiv.). Après 2 h de réaction, le milieu réactionnel est hydrolysé par addition de méthanol, puis dilué avec de l'acétate d'éthyle et lavé avec une solution aqueuse d'acide chlorhydrique pH=3 (10 ml). La phase aqueuse est séparée et basifiée à pH=10 avec de la soude, extraite avec de l'acétate d'éthyle (2x20 ml). Les phases organiques rassemblées sont séchées sur sulfate de sodium et évaporées. L'analyse RMN indique la formation majoritaire de la 4"-deoxy-4"(R)-N-benzylamino-9-deoxo-8a-aza-8a-méthyl-8a-homoérythromycine A.

### EXEMPLE 2

### Synthèse de la 4"-deoxy-4"-(R)-amino-9-deoxo-8a-aza-8a-méthyl-8a-homoérythromycine A

### 2.1/ avec le système Ti(Oi-Pr)₄/HMDS/LiBH₄:

Une solution d'isopropoxyde de titane (IV) (7,5 ml, 25,4 mmol, 4 équiv.) et d'hexaméthyldisilazane (10,5 ml, 50 mmol, 8 équiv.) est agitée 5h à température ambiante. Une solution de 2'-O-acétyl-4"-deoxy-4"-oxo-9-deoxo-8a-aza-8a-méthyl-8a-homoérythromycine A (5 g, titre= 69 % p/p, 4,4 mmol) dans du toluène (20 ml) est alors additionnée et l'ensemble est agité 4h à température ambiante puis 20 h à 70°C. Après retour à température ambiante, on additionne une solution à 10 % de borohydrure de lithium dans du THF (5 ml, 22,9 mmol, 3,6 équiv.) diluée dans du toluène (15 ml). Après 2h de réaction, on additionne du méthanol puis le milieu est dilué avec de l'acétate d'éthyle et versé sur de l'eau (50 ml). La phase aqueuse est acidifiée à pH=2-3 puis séparée après extraction et basifiée à pH=10 par addition de soude. Après extraction à l'acétate d'éthyle (2x 60 ml), les phases organiques rassemblées sont séchées sur sulfate de sodium et évaporées (4,27 g). Le brut est repris dans du méthanol (30 ml) et cette solution est chauffée 24 h à 45°C. Après évaporation du méthanol, l'analyse HPLC indique un rapport (4"*R*)/(4"S)=73/27 et un rendement de 41% pour le dérivé (4"*R*)-amino.

### 2.2/ avec le système Ti(Oi-Pr)₄/HMDS/NaBH₄:

Une solution d'isopropoxyde de titane (IV) (3,75 ml, 12,7 mmol, 2,9 équiv.) et de 2'-O-acétyl-4"-deoxy-4"-oxo-9-deoxo-8a-aza-8a-méthyl-8a-homoérythromycine A (5 g, titre= 69 % p/p, 4,4 mmol) dans du THF (15 ml) est agitée 5 h à température ambiante. De l'hexaméthyldisilazane (5,25 ml, 24,9 mmol, 5,6 équiv.) est additionné lentement à température ambiante. Le mélange réactionnel est ensuite chauffé 20 h à 55 °C. Après retour à température ambiante, on additionne par petites fractions du borohydrure de sodium (1 g, 26,4 mmol, 6 équiv.). Après 18 h d'agitation, le mélange réactionnel est versé sur de l'eau pH=3 (50 ml) et de l'acétate d'éthyle (20 ml). Après extraction, la phase aqueuse est séparée, basifiée à pH=10 et extraite avec de l'acétate d'éthyle (2x50 ml). Les phases organiques rassemblées sont séchées sur sulfate de sodium et évaporées (4,44 g). Le brut est repris dans du méthanol (30 ml) et cette solution est chauffée 24 h à 45°C. Après évaporation du méthanol, l'analyse HPLC indique un rapport (4"*R*)/(4"*S*)=76/24 et un rendement de 60% pour le dérivé (4"*R*)-amino.

### 2.3/ avec le système Al(Oi-Pr)₃/NH₃/LiBH₄:

On fait barboter pendant 10 min de l'ammoniac dans une solution toluènique (2 ml) d'isopropoxyde d'aluminium (III) (1 g, 4,9 mmol, 3,9 équiv.). On additionne ensuite une solution de 2'-O-acétyl-4"-deoxy-4"-oxo-9-deoxo-8a-aza-8a-méthyl-8a-homoérythromycine A (0,500 g, 0,63 mmol) dans du toluène (0,6 ml). Après 42 h d'agitation à température ambiante, le mélange réactionnel est refroidi à 0°C puis on additionne une solution 2M de borohydrure de lithium dans du THF (1,27 ml, 2,5 mmol, 4 équiv.). Après 5 h d'agitation, on additionne du méthanol puis le milieu est dilué avec de l'acétate d'éthyle et versé sur de l'eau (30 ml). La phase aqueuse est acidifiée à pH=2-3 puis séparée après extraction et basifiée à pH=10 par addition de soude. Après extraction à l'acétate d'éthyle (2x40 ml), les phases organiques rassemblées sont séchées sur sulfate de sodium et évaporées. Le brut est repris dans du méthanol (10 ml) et cette solution est chauffée 24 h à 45°C. Après évaporation du méthanol (0,382 g), l'analyse HPLC indique un rapport (4"*R*)/(4"*S*)=88/12 et un rendement de 56% pour le dérivé (4"R)-amino.

### 2.4/ avec le système Ti(Oi-Pr)₄/NH₃/LiBH₄:

On fait barboter pendant 30 min de l'ammoniac dans une solution d'isopropoxyde de titane (IV) (1,5 ml, 5,1 mmol, 4 équiv.). Ce mélange est ensuite transféré sur une solution de 2'-O-acétyl-4"-deoxy-4"-oxo-9-deoxo-8a-aza-8a-méthyl-8a-homoérythromycine A (1 g, titre = 72% p/p, 0,9 mmol) dans du dichlorométhane (1 ml). Après 24 h d'agitation à température ambiante, on additionne une solution à 10% (dans le THF) de borohydrure de lithium (0,93 g, 5 mmol, 4 équiv.) diluée avec du THF (1 ml). Après 2 h d'agitation, on additionne du méthanol puis le milieu est dilué avec de l'acétate d'éthyle et versé sur de l'eau (30 ml). La phase aqueuse est acidifiée à pH=2-3 puis séparée après extraction et basifiée à pH=10 par addition de soude. Après extraction à l'acétate d'éthyle (2x40 ml), les phases organiques rassemblées sont séchées sur sulfate de sodium et évaporées (0,96 g). Le brut est repris dans du méthanol (10 ml) et cette solution est chauffée 24 h à 45°C. Après évaporation du méthanol, l'analyse HPLC indique un rapport (4"*R*)/(4"*S*)=72/38 et un rendement de 73% pour le dérivé (4"R)-amino.

### 2.5/ avec le système Ti(acac)₂(Oi-Pr)₂/NH₃/LiBH₄:

On solubilise de la 4"-deoxy-4"-oxo-9-deoxo-8a-aza-8a-méthyl-8a-homoérythromycine A (100 mg, 0,13 mmol) dans une solution 2N d'ammoniac dans l'isopropanol (0,27 ml). On additionne ensuite une solution à 75% de diisopropoxyde bis(acétylacétonate) de titane dans l'isopropanol (0,13 ml, 0,27 mmol, 2 équiv.). Arès 20 h d'agitation à température ambiante, on additionne une solution 2M de borohydrure de lithium dans du THF (0,27 ml, 0,53 mmol, 4 équiv.). Après 1,5 h de réaction, on additionne du méthanol puis le milieu est dilué avec de l'acétate d'éthyle et versé sur de l'eau (20 ml). La phase aqueuse est acidifiée à pH=2-3 puis séparée après extraction et basifiée à pH=10 par addition de soude. Après extraction à l'acétate d'éthyle (2x15 ml), les phases organiques rassemblées sont séchées sur sulfate de sodium et évaporées. L'analyse HPLC indique un rapport (4"*R*)/(4"*S*)=61/39.

### • 2.6/ avec le système Zn(OCOCF₃)₂/HMDS/NaBH₄:

Un mélange de 2'-O-acétyl-4"-deoxy-4"-oxo-9-deoxo-8a-aza-8a-méthyl-8a-homoérythromycine A (100 mg, 0,13 mmol), de trifluoroacétate de zinc (43 mg, 1,4 mmol, 1,15 équiv.) et d'hexaméthyldisilazane (0,2 ml, 0,9 mmol, 7 équiv.) dans de l'acétate d'éthyle est chauffé 13 h à 60°C. Après retour à température ambiante, on additionne du borohydrure de sodium (20 mg, 0,53 mmol, 4 équiv.). Après 18 h d'agitation, le milieu est dilué avec du méthanol et agité 24 h à température ambiante. Le milieu est ensuite dilué avec de l'acétate d'éthyle (10 ml) et.versé sur de l'eau (20 ml). La phase aqueuse est acidifiée à pH=2-3 puis séparée après extraction et basifiée à pH=10 par addition de soude. Après extraction à l'acétate d'éthyle (2x15 ml), les phases organiques rassemblées sont séchées sur sulfate de sodium et évaporées. L'analyse HPLC indique un rapport (4"*R*)/(4"*S*)=50/50.

### 2.7/ avec le système Ti(Oi-Pr)₄/HMDS/(PhCO₂)₂BH₂Na:

A une solution de 2'-O-acétyl-4"-deoxy-4"-oxo-9-deoxo-8a-aza-8a-méthyl-8a-homoérythromycine A (200 mg, 0,25 mmol) dans de l'acétonitrile (2 ml) à température ambiante sont successivement rajoutés de l'hexaméthyldisilazane (0,5 ml, 2,4 mmol, 9,3 équiv.) et de l'isopropoxyde de titane (IV) (0,12 ml, 0,41 mmol, 1,6 équiv.). Après 48 h d'agitation à température ambiante, on rajoute une suspension de dibenzoyloxyborohydrure de sodium (10 équiv.) (préparé par réaction entre 1 équiv. de borohydrure de sodium et 2 équiv. d'acide benzoïque) dans du THF (1 ml). Après 18h d'agitation à température ambiante, le milieu est ensuite dilué avec de l'acétate d'éthyle (10 ml) et versé sur de l'eau (20 ml). La phase aqueuse est acidifiée à pH=2-3 puis séparée après extraction et basifiée à pH=10 par addition de soude. Après extraction à l'acétate d'éthyle (2x15 ml), les phases organiques rassemblées sont séchées sur sulfate de sodium et évaporées. L'analyse HPLC indique un rapport (4"*R*)/(4"*S*)=73/27.

### 2.8/ avec le système Ti(Oi-Pr)₄/HMDS/Zn(BH₄)₂:

A une solution de 2'-O-acétyl-4"-deoxy-4"-oxo-8a-aza-8a-méthyl-8a-homoérythromycine A (100 mg, 0,13 mmol) dans de l'acétonitrile (1 ml) à température ambiante sont successivement rajoutés de l'hexaméthyldisilazane (0,25 ml, 1,2 mmol, 9,3 équiv.) et de l'isopropoxyde de titane (IV) (60 µl, 0,20 mmol, 1,6 équiv.). Après 48 h d'agitation à température ambiante, on rajoute du borohydrure de zinc (8 équiv.). Après 18 h d'agitation à température ambiante, le milieu est ensuite dilué avec de l'acétate d'éthyle (10 ml) et versé sur de l'eau (20 ml). La phase aqueuse est acidifiée à pH=2-3 puis séparée après extraction et basifiée à pH=10 par addition de soude. Après extraction à l'acétate d'éthyle (2x15 ml), les phases organiques rassemblées sont séchées sur sulfate de sodium et évaporées. L'analyse HPLC indique un rapport (4"*R*)/(4"S)=73/27.

### 2.9/ avec le système Ti(Oi-Pr)₄/HMDS/n-pentOBH₃Na:

A une solution de 2'-O-acétyl-4"-deoxy-4"-oxo-8a-aza-8a-méthyl-8a-homoérythromycine A (100 mg, 0,13 mmol) dans de l'acétonitrile (1 ml) à température ambiante sont successivement rajoutés de l'hexaméthyldisilazane (0,25 ml, 1,2 mmol, 9,3 équiv.) et de l'isopropoxyde de titane (IV) (60 µl, 0,20 mmol, 1,6 équiv.). Après 48 h d'agitation à température ambiante, on rajoute n-pentoxyborohydrure de sodium (9 équiv.; préparé par réaction entre 1 équiv. de borohydrure de sodium et 1 équiv. de n-pentanol). Après 18 h d'agitation à température ambiante, le milieu est ensuite dilué avec de l'acétate d'éthyle (10 ml) et versé sur de l'eau (20 ml). La phase aqueuse est acidifiée à pH=2-3 puis séparée après extraction et basifiée à pH=10 par addition de soude. Après extraction à l'acétate d'éthyle (2x15 ml), les phases organiques rassemblées sont séchées sur sulfate de sodium et évaporées. L'analyse HPLC indique un rapport (4"*R*)/(4"*S*)=63/37.

### 2.10/ avec le système Ti(Oi-Pr)₄/HMDS/ LiBH₄:

Une solution d'isopropoxyde de titane (IV) (0,15 ml, 0,5 mmol, 4 équiv.) et d'hexaméthyldisilazane (0,11 ml, 0,5 mmol, 4 équiv.) est agitée 1h à température ambiante. La 2'-O-acétyl-4"-deoxy-4"-oxo-9-deoxo-8a-aza-8a-méthyl-8a-homoérythromycine A (0,100 g, 0,13 mmol) est alors additionnée et cette solution est agitée 18h à température ambiante. On additionne une solution 2M de borohydrure de lithium dans du THF (0,25 ml, 0,5 mmol, 4 équiv.). Après 4 h de réaction, on additionne du méthanol puis le milieu est dilué avec de l'acétate d'éthyle et versé sur de l'eau (10 ml). La phase aqueuse est acidifiée à pH=2-3 puis séparée après extraction et basifiée à pH=10 par addition de soude. Après extraction à l'acétate d'éthyle (2x 20 ml), les phases organiques rassemblées sont séchées sur sulfate de sodium et évaporées. Le brut est repris dans du méthanol (10 ml) et cette solution est chauffée 24 h à 45°C. Après évaporation du méthanol, l'analyse HPLC indique un rapport (4"*R*)/(4"*S*)=91/9.

## Revendications

1. Procédé de préparation d'un composé de formule générale I 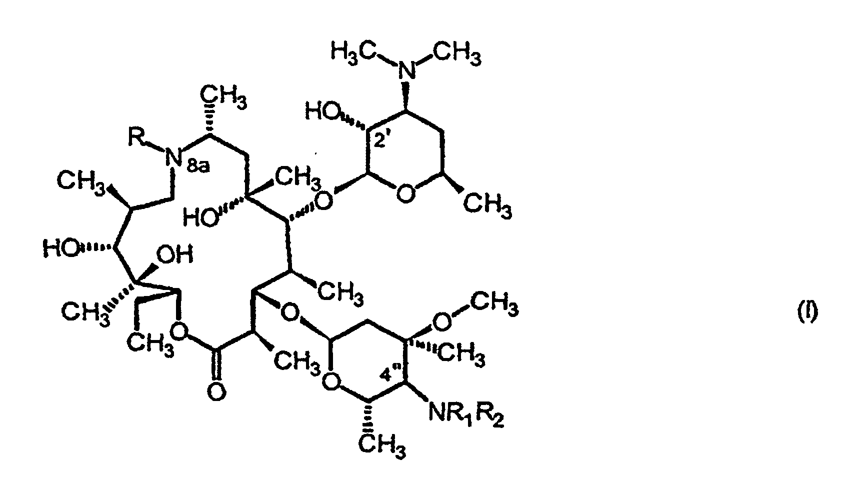 dans laquelle :
- R est un atome d'hydrogène, un groupement alkyle en C₁-C₁₀ ou arylsulfonyle en C₆-C₁₂ le cas échéant substitués et
- R₁ et R₂, identiques ou différents, représentent
• un atome d'hydrogène,
• un groupement alkyle en C₁-C₁₀ éventuellement substitué par un ou plusieurs groupements aryle en C₆-C₁₂ le cas échéant substitué,
par amination réductrice d'un composé de formule générale II : 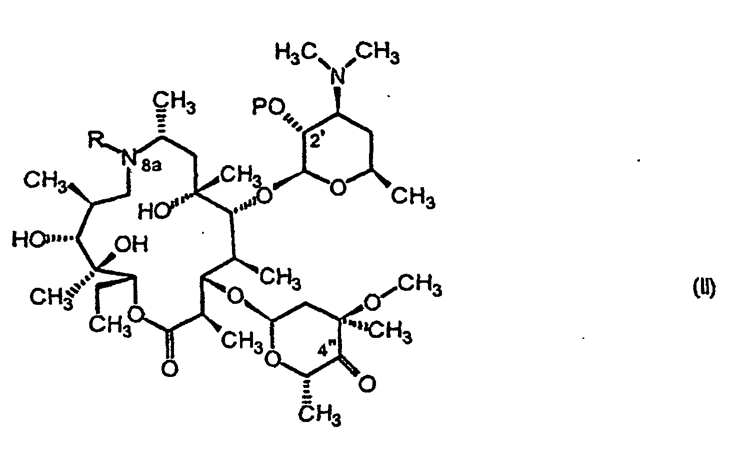
- avec R tel que défini en formule générale I, et
- P représentant un atome d'hydrogène ou un groupement protecteur,
**caractérisé en ce qu'**il comprend :
- la mise en présence dudit composé de formule générale II avec au moins un réactif azoté et un acide de Lewis aprotique dans des conditions favorables à la transformation de la fonction carbonyle 4",
- la réduction du milieu résultant à l'aide d'un agent réducteur, et
- éventuellement la déprotection de la fonction hydroxyle en position 2',
pour conduire au composé de formule générale I attendu.

2. Procédé selon la revendication 1, **caractérisé en ce que** le composé de formule générale I est obtenu sous la forme d'un mélange de ses 2 isomères 4"R et 4"S.

3. Procédé selon la revendication 1 ou 2, **caractérisé en ce que** l'isomère 4"R est obtenu majoritairement.

4. Procédé selon l'une des revendications 1 ou 2, **caractérisé en ce que** le réactif azoté est choisi parmi l'ammoniac, les sels d'ammonium comme l'acétate d'ammonium, le chlorhydrate d'ammonium, les amines primaires NH₂R_{A}, les amines secondaires NH(R_{A})₂ avec les radicaux R_{A}, identiques ou différents, représentent un groupement alkyle en C₁ à C₁₀, aryle en C₆ à C₁₂ ou silyle de type SiR_{B}R_{C}R_{D} dans lequel les groupements R_{B}, R_{C} et R_{D} identiques ou différents, peuvent être un groupement alkyle ou aryle.

5. Procédé selon la revendication 4, **caractérisé en ce que** le réactif azoté est choisi parmi la benzylamine, l'hexaméthyldisilazane et l'ammoniac.

6. Procédé selon l'une des revendications précédentes, **caractérisé en ce que** le réactif azoté est introduit à raison de 1 à 30 équivalents par rapport au composé de formule générale II et de préférence à raison de 1 à 10 équivalents.

7. Procédé selon l'une des revendications précédentes, **caractérisé en ce que** l'acide de Lewis est introduit à raison de 1 à 30 équivalents par rapport au composé de formule générale II.

8. Procédé selon l'une des revendications précédentes, **caractérisé en ce que** l'acide de Lewis est choisi parmi les complexes organométalliques des éléments de la colonne IVB, IIIA ou IIB du tableau de la classification périodique, dont notamment le titane, le zinc ou l'aluminium.

9. Procédé selon la revendication 8, **caractérisé en ce que** ledit complexe est choisi parmi l'isopropoxyde de titane (IV), l'isopropoxyde d'aluminium (III), l'isopropoxyde bis (acétyl acétonate) de titane (IV),le trifluoroacétate de zinc (II).

10. Procédé selon l'une des revendications précédentes, **caractérisé en ce que** l'agent réducteur est un hydrure métallique.

11. Procédé selon la revendication 10, **caractérisé en ce que** l'agent réducteur est un hydrure métallique.

12. Procédé selon l'une des revendications 10 ou 11, **caractérisé en ce qu'**il s'agit du borohydrure de sodium, de lithium ou de zinc.

13. Procédé selon l'une quelconque des revendications précédentes, **caractérisé en ce qu'**il est mis en oeuvre en présence d'un solvant organique choisi parmi les hydrocarbures aromatiques, notamment le toluène, les solvants halogénés comme le dichlorométhane, les alcools comme le méthanol, les nitriles comme l'acétonitrile, les éthers comme le THF ou les sulfoxydes, comme le DMSO.

14. Procédé selon l'une quelconque des revendications précédentes, **caractérisé en ce que** l'acide de Lewis est l'isopropoxyde de titane ou l'isopropoxyde d'aluminium, le réactif azoté est l'ammoniac, l'hexaméthyldisilazane ou la benzylamine, et le solvant est le dichlorométhane, le tétrahydrofurane ou le toluène.

15. Procédé selon la revendication 14, **caractérisé en ce que** l'agent réducteur est le borohydrure de sodium ou de lithium.

16. Procédé selon l'une quelconque des revendications précédentes, **caractérisé en ce que** le composé de formule II en solution dans un solvant organique est ajouté au mélange de l'acide de Lewis et du réactif azoté.

17. Procédé selon l'une quelconque des revendications 1 à 15, **caractérisé en ce que** le composé de formule II et l'acide de Lewis sont mélangés dans un solvant organique et on ajoute lentement le réactif azoté à ce mélange.

18. Procédé selon l'une quelconque des revendications 1 à 15, **caractérisé en ce que** le composé de formule II est mélangé avec le réactif azoté dans un solvant organique et on ajoute ensuite l'acide de Lewis.

## Patentansprüche

1. Verfahren zur Herstellung einer Verbindung der allgemeinen Formel I 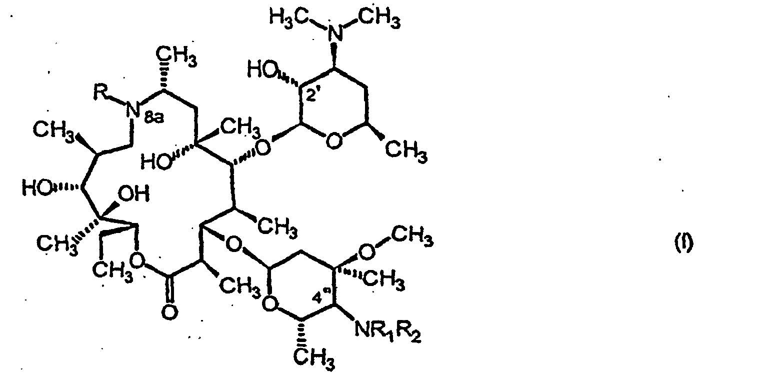 wobei:
R ein Wasserstoffatom, ein gegebenenfalls substituierter C₁-C₁₀-Alkylrest oder ein gegebenenfalls substituierter C₆-C₁₂-Arylsulfonylrest ist und
R₁ und R₂, gleich oder voneinander verschieden, ein Wasserstoffatom, einen C₁-C₁₀-Alkylrest, gegebenenfalls substituiert mit einem oder mehreren gegebenenfalls substituierten Arylresten, oder einen gegebenenfalls substituierten C₆-C₁₂-Arylrest bedeuten,
durch reduktive Aminierung einer Verbindung der allgemeinen Formel II: 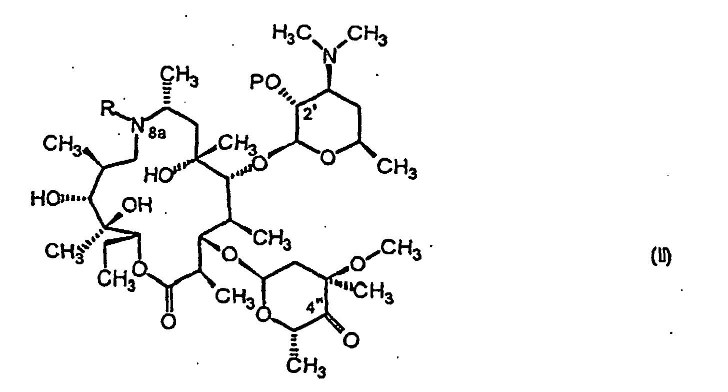 wobei R wie in der allgemeinen Formel I definiert ist und
P ein Wasserstoffatom oder eine Schutzgruppe darstellt,
**dadurch gekennzeichnet, daß** es umfaßt:
Bereitstellen der Verbindung der allgemeinen Formel II mit mindestens einem Stickstoff-Reagens und einer aprotischen Lewis-Säure unter fiir die Umwandlung der
Carbonylfunktion 4" günstigen Bedingungen,
Reduktion des resultierenden Mediums mit Hilfe eines Reduktionsmittels und gegebenenfalls Entfernen der Schutzgruppe aus der Hydroxylfunktion an Position 2', um die erwartete Verbindung der allgemeinen Formel I zu erhalten.

2. Verfahren nach Anspruch 1, **dadurch gekennzeichnet, daß** die Verbindung der allgemeinen Formel I in Form eines Gemisches ihrer zwei Isomere 4"R und 4"S erhalten wird.

3. Verfahren nach Anspruch 1 oder 2, **dadurch gekennzeichnet, daß** überwiegend das Isomer 4"R erhalten wird.

4. Verfahren nach einem der Ansprüche 1 oder 2, **dadurch gekennzeichnet, daß** das Stickstoff-Reagens aus Ammoniak, Ammoniumsalzen, wie Ammoniumacetat, Ammoniumchlorhydrat, primären Aminen NH₂R_{A}, sekundären Aminen NH(R_{A})₂ ausgewählt ist, wobei die Reste R_{A}, gleich oder voneinander verschieden, einen C₁-C₁₀-Alkylrest, einen C₆-C₁₂-Arylrest oder Silyl vom Typ SiR_{B}R_{C}R_{D} bedeuten, wobei die Reste R_{B}, R_{C} und R_{D}, gleich oder voneinander verschieden, ein Alkyl- oder Arylrest sein können.

5. Verfahren nach Anspruch 4, **dadurch gekennzeichnet, daß** das Stickstoff-Reagens aus Benzylamin, Hexamethyldisilazan und Ammoniak ausgewählt ist.

6. Verfahren nach einem der vorhergehenden Ansprüche, **dadurch gekennzeichnet, daß** das Stickstoff-Reagens in einem Verhältnis von 1 bis 30 Äquivalenten, bezogen auf die Verbindung der allgemeinen Formel II, und vorzugsweise in einem Verhältnis von 1 bis 10 Äquivalenten zugegeben wird.

7. Verfahren nach einem der vorhergehenden Ansprüche, **dadurch gekennzeichnet, daß** die Lewis-Säure in einem Verhältnis von 1 bis 30 Äquivalenten, bezogen auf die Verbindung der allgemeinen Formel II, zugegeben wird.

8. Verfahren nach einem der vorhergehenden Ansprüche, **dadurch gekennzeichnet, daß** die Lewis-Säure aus Organometall-Komplexen der Elemente der Gruppe IVB, IIIA oder IIB des Periodensystems, insbesondere Titan, Zink oder Aluminium, ausgewählt ist.

9. Verfahren nach Anspruch 8, **dadurch gekennzeichnet, daß** der Komplex aus Titan(IV)isopropoxid, Aluminium(III)isopropoxid, Titan(IV)bis(acetylacetonat)-isopropoxid und Zink(II)trifluoracetat ausgewählt ist.

10. Verfahren nach einem der vorhergehenden Ansprüche, **dadurch gekennzeichnet, daß** das Reduktionsmittel ein metallisches Hydrid ist.

11. Verfahren nach Anspruch 10, **dadurch gekennzeichnet, daß** das Reduktionsmittel ein metallisches Hydrid ist.

12. Verfahren nach einem der Ansprüche 10 oder 11, **dadurch gekennzeichnet, daß** es sich um Natrium-, Lithium- oder Zinkborhydrid handelt.

13. Verfahren nach einem der vorhergehenden Ansprüche, **dadurch gekennzeichnet, daß** es in Gegenwart eines organischen Lösungsmittels, ausgewählt aus aromatischen Kohlenwasserstoffen, insbesondere Toluol, halogenierten Lösungsmitteln wie Dichlormethan, Alkoholen wie Methanol, Nitrilen wie Acetonitril, Ethern wie THF, oder Sulfoxiden wie DMSO, durchgeführt wird.

14. Verfahren nach einem der vorhergehenden Ansprüche, **dadurch gekennzeichnet, daß** die Lewis-Säure Titanisopropoxid oder Aluminiumisopropoxid ist, das Stickstoff-Reagens Ammoniak, Hexamethyldisilazan oder Benzylamin ist, und das Lösungsmittel Dichlormethan, Tetrahydrofuran oder Toluol ist.

15. Verfahren nach Anspruch 14, **dadurch gekennzeichnet, daß** das Reduktionsmittel Natrium- oder Lithiumborhydrid ist.

16. Verfahren nach einem der vorhergehenden Ansprüche, **dadurch gekennzeichnet, daß** die Verbindung der Formel II in Lösung in einem organischen Lösungsmittel dem Gemisch der Lewis-Säure und des Stickstoff-Reagens zugegeben wird.

17. Verfahren nach einem der Ansprüche 1 bis 15, **dadurch gekennzeichnet, daß** die Verbindung der Formel II und die Lewis-Säure in einem organischen Lösungsmittel gemischt werden und man diesem Gemisch langsam das Stickstoff-Reagens zugibt.

18. Verfahren nach einem der Ansprüche 1 bis 15, **dadurch gekennzeichnet, daß** die Verbindung der Formel II mit dem Stickstoff-Reagens in einem organischen Lösungsmittel gemischt wird und man anschließend die Lewis-Säure zugibt.

## Claims

1. A process for preparing a compound of formula I 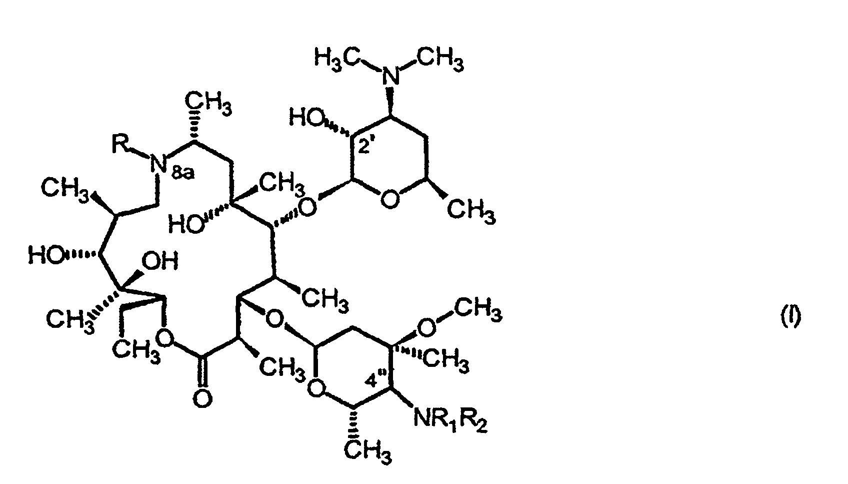 in which:
- R is a hydrogen atom or an optionally substituted C₁-C₁₀ alkyl, C₂-C₁₀ alkenyl or C₆-C₁₂ arylsulphonyl group, and
- R₁ and R₂, which may be identical or different, represent
■ a hydrogen atom, or
■ a C₁-C₁₀ alkyl group optionally substituted with one or more optionally substituted aryl groups, or an optionally substituted C₆-C₁₂ aryl group,
said process comprising a reductive amination of a compound of formula II: 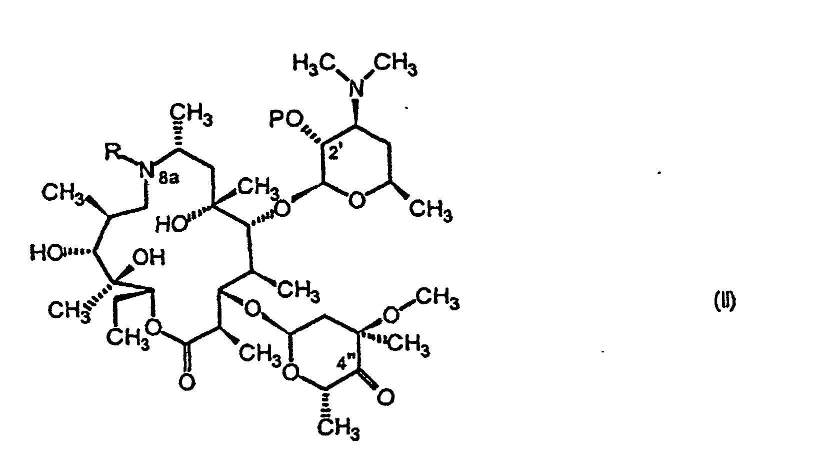 in which:
- R is as defined in the formula I, and
- P represents a hydrogen atom or a protecting group,
wherein said reductive amination comprises:
- contacting the compound of formula II with at least one nitrogenous reagent and an aprotic Lewis acid to form a reaction medium, and,
- reducing the reaction medium using a reducing agent, and
- optionally deprotecting the hydroxyl function in position 2',
to obtain said compound of the expected formula I.

2. The process according to claim 1, wherein the compound of formula I is obtained in the form of a mixture of its two isomers 4"R and 4"S.

3. The process according to any one of claims 1 to 2, wherein the 4"R isomer is obtained as the major product.

4. The process according to any one of claims 1 to 3, wherein the nitrogenous reagent is selected from the group consisting of ammonia, ammonium salt such as ammonium acetate or ammonium hydrochloride, the primary amine NH₂R_{A}, the secondary amine NH(R_{A})₂ with the radical R_{A} which may be identical or different in the secondary amine, represents a C₁ to C₁₀ alkyl, or C₆ to C₁₂ aryl group, or a silyl group of the type SiR_{B}R_{C}R_{D} in which the groups R_{B}, R_{C} and R_{D}, which may be identical or different, represent an alkyl or aryl group.

5. The process according to claim 4, wherein the nitrogenous reagent is selected from the group consisting of benzylamine, hexamethyldisilazane and ammonia.

6. The process according to any one of claims 1 to 5, wherein the nitrogenous reagent is introduced in a proportion of from 1 to 30 equivalents relative to the compound of formula II, and preferably from 1 to 10 equivalents relative to the compound of formula II.

7. The process according to any one of claims 1 to 6, wherein the Lewis acid is introduced in a proportion of from 1 to 30 equivalents relative to the compound of formula II.

8. The process according to any one of claims 1 to 7, wherein the Lewis acid is an organometallic complex wherein to metal of the organometallic complex is an element from column IVB, IIIA or IIB of the Periodic Table of the Elements, preferably titanium, zinc or aluminum.

9. The process according to claim 8, wherein the organometallic complex is selected from the group consisting of titanium(IV) isopropoxide, aluminium(III) isopropoxide, titanium(IV) isopropoxide bis(acetylacetoflate) and zinc(II) trifluoroacetate.

10. The process according to any one of claims 1 to 9, wherein the reducing agent is a metal hydride.

11. The process according to claim 10, wherein the reducing agent is a boron or aluminium hydride.

12. The process according to any one of claims 10 or 11, wherein the reducing agent is sodium, lithium or zinc borohydride.

13. The process according to any one of claims 1 to 12, wherein the process is performed in the presence of an organic solvent selected from the group consisting of the aromatic hydrocarbons, such as toluene, the halogenated organic solvents such as dichloromethane, the alcohols such as methanol, the nitriles such as acetonitrile, the ethers such as tetrahydrofuran and the sulphoxides such as DMSO.

14. The process according to any one of the claims 1 to 13, wherein the Lewis acid is titanium isopropoxide or aluminium isopropoxide, the nitrogenous reagent is ammonia, hexamethyldisilazane or benzylamine, and the solvent is dichloromethane, tetrahydrofuran or toluene.

15. The process according to claim 14, wherein the reducing agent is sodium borohydride or lithium borohydride.

16. The process according to any one of claims 1 to 15, wherein the compound of formula II dissolved in an organic solvent is added to a mixture of the Lewis acid and the nitrogenous reagent.

17. The process according to any one of claims 1 to 15, wherein the compound of formula II and the Lewis acid are mixed in an organic solvent and the nitrogenous reagent is slowly added to the mixture.

18. The process according to any one of claims 1 to 15, wherein the compound of formula II is mixed to the nitrogenous reagent in an organic solvent, and the Lewis acid is added to the mixture.
